# EUROPEAN PATENT SPECIFICATION

(11) **EP 4 414 461 B1**
(45) Date of publication and mention of the grant of the patent: **01.04.2026**
(21) Application number: 23156142.4
(22) Date of filing: 10.02.2023
(51) Int. Cl.: C12Q 1/6886, C12Q 1/689

(54) **METHOD OF DETECTING AN INCREASED RISK OF HAVING OR DEVELOPING RCC**
VERFAHREN ZUR ERKENNUNG EINES ERHÖHTEN RISIKOS, RCC ZU HABEN ODER ZU ENTWICKELN
PROCÉDÉ DE DÉTECTION D'UN RISQUE ACCRU D'AVOIR OU DE DÉVELOPPER UNE RCC

(43) Date of publication of application: 14.08.2024
(73) Proprietor: Trustlife Therapeutics Inc., San Diego, CA 92101 (US)
(72) Inventor: MARDINOGLU, Adil, 412 60 Göteborg (SE); SHOAIE, Saeed, Cambridge, CB2 9AX (GB); PORTLOCK, Theo John, Derbyshire, DE56 1AP (GB)
(74) Representative: Kransell & Wennborg KB

(56) References cited:
- WO-A1-2022/157207
- US-A1- 2022 056 532
- WANG JUNPENG ET AL: "Uncovering the microbiota in renal cell carcinoma tissue using 16S rRNA gene sequencing", JOURNAL OF CANCER RESEARCH AND CLINICAL ONCOLOGY, vol. 147, no. 2, 21 November 2020 (2020-11-21), pages 481 - 491, XP037341797, ISSN: 0171-5216, DOI: 10.1007/S00432-020-03462-W
- SONG MINGYANG ET AL: "Influence of the Gut Microbiome, Diet, and Environment on Risk of Colorectal Cancer", GASTROENTEROLOGY, ELSEVIER INC, US, vol. 158, no. 2, 3 October 2019 (2019-10-03), pages 322 - 340, XP085976543, ISSN: 0016-5085, [retrieved on 20191003], DOI: 10.1053/J.GASTRO.2019.06.048

## Description

### TECHNICAL FIELD

The present disclosure relates to the field of cancer screening and in particular renal cell carcinoma (RCC) screening.

### BACKGROUND

Worldwide, there are over 400,000 new cases of RCC and over 170,000 deaths annually due to kidney cancer (https://gco.iarc.fr). To date the gut microbiome has not been connected to RCC.

RCC typically is diagnosed through imaging studies such as a computed tomography (CT) scan with contrast or an ultrasound, or a combination of imaging modalities. These imaging tests help the physician identify any abnormal or suspicious masses that could be a sign of carcinoma.

In addition to imaging tests, a sample of the abnormal cells may be obtained with a needle biopsy procedure to confirm the diagnosis. During a needle biopsy, a needle is inserted through the skin and into the renal mass. This procedure allows the physician to extract a sample of cells, which can be examined under a microscope for definitive diagnosis.

Other tests that may be used in the diagnosis of renal carcinoma include a urinalysis, blood tests, and a chest X-ray. These tests help the physician determine the health of the other organs in the body and identify any other signs of the disease.

Mohan et al. (Syst Appl Microbiol. 2006 Jun;29(4):292-9) isolated an obligatory anaerobic, Gram-positive, rod-shaped organism from feces of a healthy human donor and proposed to classify this novel bacterium as a new species, *Clostridium asparagiforme.* The bacterium has later been reclassified as *Enterocloster asparagiformis* (Haas and Blanchard, Int J Syst Evol Microbiol 2020;70:23-34).

### SUMMARY

The present disclosure is based on an analysis of the association of bacteria with the classification of RCC using interpretable machine learning methodologies.

A machine learning classification model has been trained, tested and interpreted to discriminate RCC from healthy samples using bacterial species abundance data and a 31 species signature for RCC has been characterized. Of those species, enrichment of *Enterocloster asparagiformis* was uniquely predictive of RCC as compared with other disease signatures.

Hence the present disclosure provides a method of detecting an increased risk of having or developing renal cell carcinoma (RCC), comprising the steps of:
a) determining the level of *Enterocloster asparagiformis* in a fecal sample from a human subject;
b) comparing the determined level to a reference level; and
c) if the determined level is higher than the reference level, concluding that the human subject has the increased risk of having or developing RCC.

### BRIEF DESCRIPTION OF THE DRAWINGS

Figure 1 is a PCoA scatterplot showing difference in beta diversity between PRJEB22863 RCC and PRJEB6070 healthy samples.
Figure 2 is an AUCROC curve showing high prediction scoring between classification of RCC and healthy samples (positive label is RCC).
Figure 3 describes the 31 species signature of RCC. It is a bar plot showing SHAP scores for the prediction of RCC (positive and negative values indicates an increase and decrease in abundance is predictive of RCC respectively) filtered by the mean directional shap score of above 0.004. In bold is *Enterocloster asparagiformis:* a species enrichment unique to RCC.
Figure 4 shows the most explainable species for the classification of RCC. Left: Stripplot of relative abundance of the depletion signature species *Dorea formicigenerans* (FDR adjusted M.W.W. = 7.11e-11). Right: Stripplot of relative abundance of enrichment signature species *Enterocloster asparagiformis* (FDR adjusted M.W.W. = 8.54e-7).
Figure 5 shows a comparison of the log2 fold change of RCC to Healthy ('Log(RCC/Healthy') to its significance (M.W.W. FDR adjusted - annotated as 'Log10(FDR)') with the SHAP score for each species denoted as the point size. Annotated point is *Enterocloster asparagiformis.*

### DETAILED DESCRIPTION

The present disclosure provides a method of detecting an increased risk of having or developing renal cell carcinoma (RCC), comprising the steps of:
a) determining the level of *Enterocloster asparagiformis* in a fecal sample from a human subject;
b) comparing the determined level to a reference level; and
c) if the determined level is higher than the reference level, concluding that the human subject has the increased risk of having or developing RCC.

As understood by the skilled person, this method is typically carried out ex *vivo.*

The reference level of the method is for example a predetermined level based on determinations of the level of *Enterocloster asparagiformis* in fecal samples from human subjects not suffering from RCC, such as healthy human subjects and/or human subjects suffering from another cancer than RCC.

In one embodiment, the method further comprises a step of further examining the human subject having the increased risk of having or developing RCC to establish an RCC diagnosis. Such a step of further examination may comprise microscopic examination of cells from a biopsy and/or imaging.

Step a) may comprise quantitative real-time PCR (qPCR). To facilitate the qPCR, bacterial DNA is typically extracted from the fecal sample. A primer specific for *Enterocloster asparagiformis* is then used.

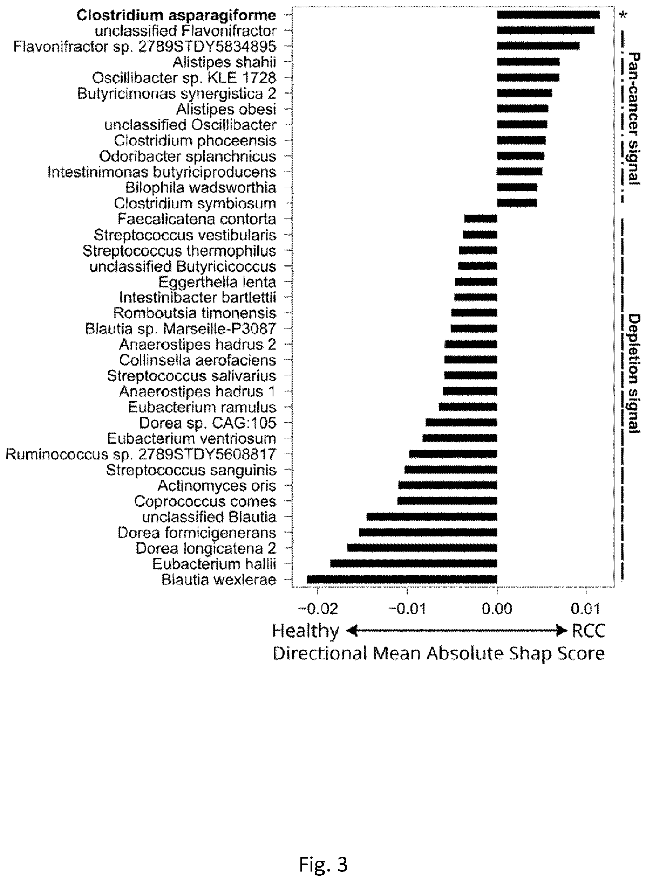

In one embodiment, the level of a further bacterium (such as another bacterium having a positive directional mean absolute shap score in Figure 3) is determined in step a), this level is compared to a relevant reference level in step b) and it is concluded in step c) that that the human subject has the increased risk of having or developing RCC if the level of *Enterocloster asparagiformis* and the level of the further bacterium are above their respective reference levels.

In another embodiment, the levels of at least two further bacteria (such as other bacteria having a positive directional mean absolute shap score in Figure 3) are determined in step a), these levels are compared to relevant reference levels in step b) and it is concluded in step c) that that the human subject has the increased risk of having or developing RCC if the level of *Enterocloster asparagiformis* and the levels of the further bacteria are above their respective reference levels.

### EXAMPLE

### Materials and methods

### Metagenomic sequencing data collection

Metagenomic species abundance data and metadata was obtained from [explain the source of the info in the Microboime Atlas.

Annotation of MSP with taxonomic information was performed using pandas in python using the database of the ICG2 mappings found at https://entrepot.recherche.data.gouv.fr/dataset.xhtml?persistentId=doi:10.15454/F LANUP.

### Statistical analysis

Difference between species abundances was calculated using the python scipy package, and the p-values were adjusted using the Benjamini-Hochberg multiple testing correction where the significance was denoted as a q-value below 0.05.

PERMANOVA p-values were calculated from Bray-Curtis Dissimilarities using the 'permanova' function from skbio.stats.distance.

### Machine learning and model interrogation

All machine learning was done using python packages. MSP species abundances were normalized using the 'StandardScaler' function from sklearn.preprocessing package before feature selection and machine learning analysis.

After preprocessing, whole dataset was split into 70% train and 30% test set using the 'train_test_split' from sklearn.model_selection.

RandomForestClassifier was the model used from sklearn.ensemble with the hyperparameters listed in Table 1.

**Table 1. Hyperparameter tuning for the Random Forest classification model.**

| PARAMETER | JUSTIFICATION |
|---|---|
| n_estimators=500 | Best performance after tuning |
| random_state=1 | Fix random state to ensure consistent results |
| n_jobs=-1 | To increase performance by using all available cores |

Model metrics were calculated with sklearn.metrics.

The 'shap' package was used for model interrogation and interpretation.

Species with a mean absolute SHAP score of above 0.004 were selected as members of the disease signature.

### Results

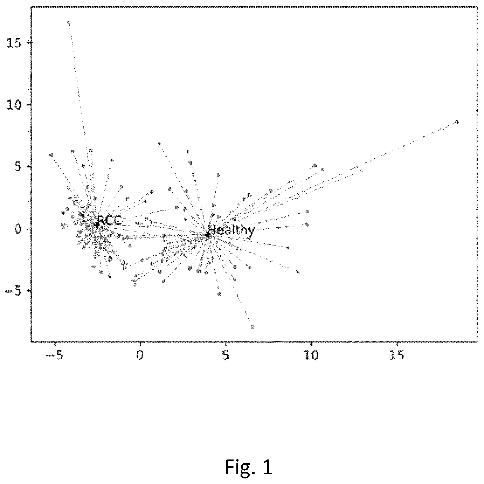

There was no matching healthy control from PRJEB22863. However, using healthy samples from matched geography cohort PRJEB6070 (French CRC cohort), we found that there was significant difference in Bray-Curtis beta diversity between the two groups (PERMANOVA p=0.001) (Fig 1).

Random Forest models from Python Scikitlearn module were trained with the hyperparameters listed in Table 1.

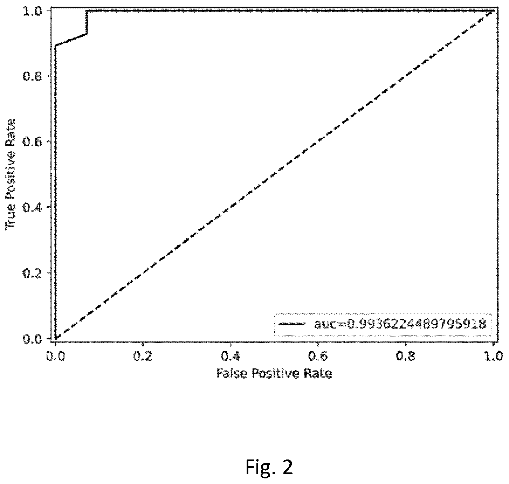

These models were highly predictive of RCC with an AUCROC of 0.994 (Fig. 2).

SHAP scoring of the species features of the model was able to classify species biomarkers of RCC (Fig. 3).

There were 31 species with mean absolute SHAP scores above 0.004, namely the following: *Butyricimonas synergistica 2, Romboutsia timonensis, Collinsella aerofaciens, Streptococcus vestibularis, Intestinimonas butyriciproducens, Dorea longicatena 2, Blautia obeum, Coprobacillus sp. CAG:235*, *Alistipes shahii, Odoribacter splanchnicus, Gemella sanguinis, Ruminococcus bromii 2, Intestinibacter bartlettii, unclassified Butyricicoccus, Dorea sp. CAG:105, Enterocloster asparagiformis* (formerly *Clostridium asparagiforme*), *Blautia massiliensis, Coprococcus catus, Blautia sp. 2789STDY5608844, Eubacterium ventriosum, Ruminococcus sp. 2789STDY5608882, Eubacterium ramulus, Streptococcus salivarius, Actinomyces oris, Streptococcus sanguinis, Anaerostipes hadrus 1, Coprococcus comes, Ruminococcus sp. 2789STDY5608817, Blautia wexlerae, Eubacterium hallii, and Dorea formicigenerans.*

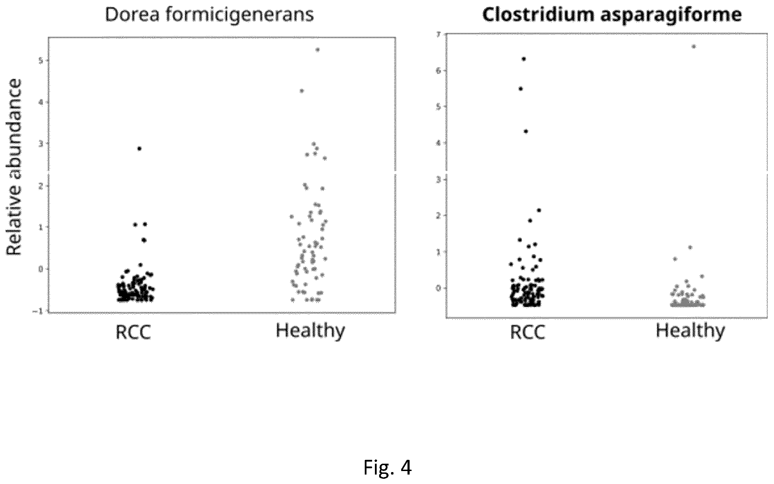

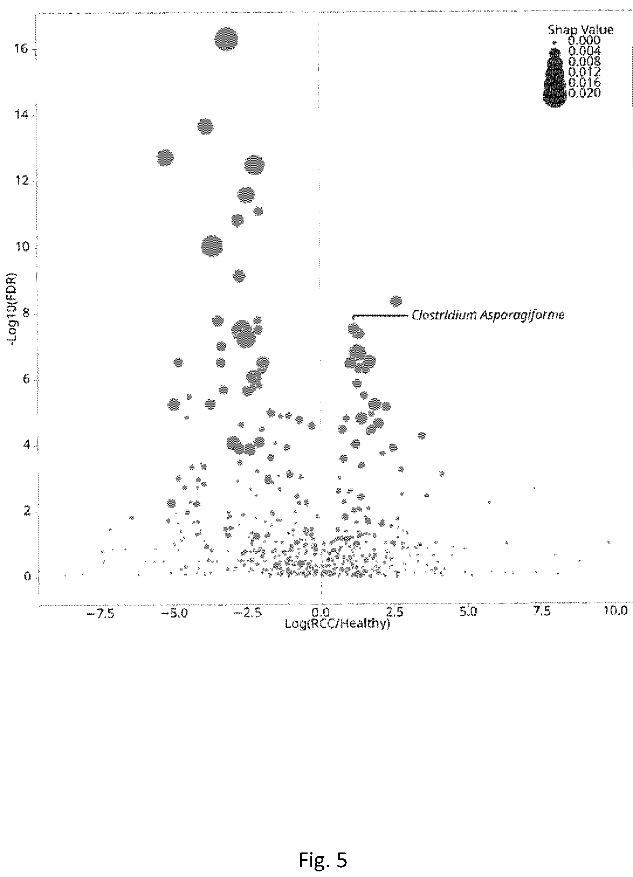

Those species were significantly differentially abundant between RCC and healthy samples (Fig. 4 and 5).

Those species where subclassified as the depletion signal (depletion of which is predictive of multiple diseases) and the pan-cancer signal (enrichment of which is predictive of multiple cancers).

Enrichment in one of those species, namely *Enterocloster asparagiformis* (formerly *Clostridium Asparagiforme*)*,* uniquely predicts RCC.

## Claims

1. A method of detecting an increased risk of having or developing renal cell carcinoma (RCC), comprising the steps of:
a) determining the level of *Enterocloster asparagiformis* in a fecal sample from a human subject;
b) comparing the determined level to a reference level; and
c) if the determined level is higher than the reference level, concluding that the human subject has the increased risk of having or developing RCC.

2. The method of claim 1, wherein the reference level is a predetermined level based on determinations of the level of *Enterocloster asparagiformis* in fecal samples from human subjects not suffering from RCC, such as healthy human subjects and/or human subjects suffering from another cancer than RCC.

3. The method of claim 1 or 2, further comprising the step of further examining the human subject having the increased risk of having or developing RCC to establish an RCC diagnosis.

4. The method of any one of the preceding claims, wherein step a) comprises quantitative real-time PCR (qPCR).

## Patentansprüche

1. Verfahren zum Nachweis eines erhöhten Risikos, an Nierenzellkarzinom (RCC) zu erkranken oder dieses zu entwickeln, umfassend die folgenden Schritte:
a) Bestimmen des Gehalts an *Enterocloster asparagiformis* in einer Stuhlprobe eines menschlichen Subjekts;
b) Vergleichen des bestimmten Gehalts mit einem Referenzgehalt; und
c) wenn der bestimmte Gehalt höher als der Referenzgehalt ist, Schlussfolgern, dass das menschliche Subjekt ein erhöhtes Risiko hat, an RCC zu erkranken oder dieses zu entwickeln.

2. Verfahren nach Anspruch 1, wobei der Referenzgehalt ein vorbestimmter Gehalt ist, der auf Bestimmungen des Gehalts an *Enterocloster asparagiformis* in Stuhlproben von menschlichen Subjekten basiert, die nicht an RCC leiden, wie beispielsweise gesunde menschliche Subjekte und/oder menschliche Subjekte, die an einer anderen Krebsart als RCC leiden.

3. Verfahren nach Anspruch 1 oder 2, das ferner den Schritt umfasst, das menschliche Subjekt mit erhöhtem Risiko, an RCC zu erkranken oder dieses zu entwickeln, weiter zu untersuchen, um eine RCC-Diagnose zu stellen.

4. Verfahren nach einem der vorstehenden Ansprüche, wobei Schritt a) eine quantitative Echtzeit-PCR (qPCR) umfasst.

## Revendications

1. Procédé de détection d'un risque accru d'avoir ou de développer un carcinome cellulaire rénal (CCR), comprenant les étapes de :
a) détermination du niveau *d'enterocloster asparagiformis* dans un échantillon fécal d'un sujet humain ;
b) comparaison du niveau déterminé avec un niveau de référence ; et
c) si le niveau déterminé est supérieur au niveau de référence, conclusion que le sujet humain présente le risque accru d'avoir ou de développer un CCR.

2. Procédé selon la revendication 1, dans lequel le niveau de référence est un niveau prédéterminé basé sur la détermination du niveau d*'enterocloster asparagiformis* dans des échantillons fécaux de sujets humains ne souffrant pas de CCR, comme de sujets humains sains et/ou de sujets humains souffrant d'un cancer autre que le CCR.

3. Procédé selon la revendication 1 ou 2, comprenant en outre l'étape consistant à continuer à examiner le sujet humain présentant le risque accru d'avoir ou de développer un CCR pour établir un diagnostic de CCR.

4. Procédé selon l'une quelconque des revendications précédentes, dans lequel l'étape a) comprend une PCR quantitative en temps réel (qPCR).
